(19) Europäisches Patentamt
European Patent Office
Office européen des brevets

(11) Publication number: **0 498 507 A1**

(12) **EUROPEAN PATENT APPLICATION**

(21) Application number: **92200311.6**

(51) Int. Cl.$^5$: **C07C 231/18**

(22) Date of filing: **04.02.92**

(30) Priority: **05.02.91 DE 4103759**

(43) Date of publication of application:
**12.08.92 Bulletin 92/33**

(84) Designated Contracting States:
**AT BE CH DE ES FR GB IT LI NL SE**

(71) Applicant: **DSM N.V.**
**Het Overloon 1**
**NL-6411 TE Heerlen(NL)**

(72) Inventor: **Oehme, Gunther**
**Augustenstrasse 127**
**O-2500 Rostock 1(DE)**
Inventor: **Petzold, Eckard**
**Usedomerstrasse 5**
**O-2520 Rostock 22(DE)**
Inventor: **Selke, Rüdiger**
**St. Georgstrasse 40**
**O-2500 Rostock 1(DE)**

(74) Representative: **Jacobs, Monique S.N. et al**
**Octrooibureau DSM Postbus 9**
**NL-6160 MA Geleen(NL)**

# (54) Process for the preparation of enantiomers of alpha-amino acid derivatives by asymmetric hydrogenation.

(57) The inventor relates to a process for the preparation of (S)- and (R)-$\alpha$-amino acid dervatives by asymmetric hydrogenation starting from a substrate of the formula I

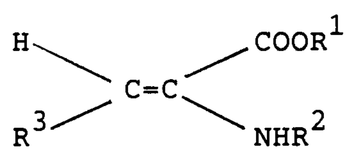

I

in which
- $R^1$ denotes hydrogen or an alkyl group
- $R^2$ denotes a protecting group and
- $R^3$ denotes hydrogen, an alkyl, aryl, aralkyl, alkaryl, heteroalkyl or heteroaryl group,

which is hydrogenated in the presence of a rhodium(I)complex catalyst of the formula II

$/Rh(Z)(L_k)/^+ A^-$ II

in which
- Rh denotes rhodium
- Z denotes a chiral chelating ligand with two trivalent phosphorous atoms as coordination partners for a bond to rhodium
- L denotes a solvent molecule
- k denotes 0, 1, 2, or 3 and
- $A^-$ denotes an anion of a weakly coordinating acid, such as $BF_4^-$, $ClO_4^-$, $SO_4H^-$, $PF_6^-$, $R^4$-$COO^-$, or $R^4SO_3^-$, where $R^4$ denotes a fluorinated alkyl or aryl group

into an $\alpha$-amino acid derivative of the formula III

EP 0 498 507 A1

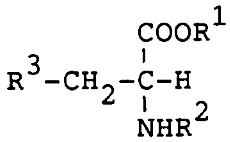 III

in which

- $R^1$ denotes hydrogen or an alkyl group
- $R^2$ denotes a protecting group and
- $R^3$ denotes hydrogen, an alkyl, aryl, aralkyl, alkaryl, heteroalkyl or heteroaryl group

in which process an amphiphilic substance of the formula IV

A-E IV

in which

- A denotes a nonpolar alkyl chain with 5 to 20 carbon atoms or a glycerol dialkylether group and
- E denotes a polar (hydrophilic)group, such as $-O-SO_3Na$, $-SO_3Na$, $-O(CH_2CH_2O)_nH$ or $-OP^{\ominus}O_2/OCH_2CH_2N^{\oplus}(CH_3)_3/$, where n is a number between 3 and 15.

The hydrogenation can be carried out in a polar or nonpolar solvent.

The invention relates to a process for the preparation of enantiomers of α-amino acid derivatives by asymmetric hydrogenation starting from a substrate of the formula I

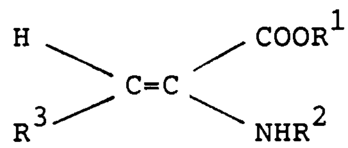

I

in which

- $R^1$ denotes hydrogen or an alkyl group
- $R^2$ denotes a protecting group and
- $R^3$ denotes hydrogen, an alkyl, aryl, aralkyl, alkaryl, heteroalkyl or heteroaryl group,

which is hydrogenated in the presence of a rhodium(I)complex catalyst of the formula II

$/Rh(Z)(L_k)/^{+} A^{-}$ II

in which

- Rh denotes rhodium
- Z denotes a chiral chelating ligand with two trivalent phosphorous atoms as coordination partners for a bond to rhodium
- L denotes a solvent molecule
- k denotes 0, 1, 2, or 3 and
- $A^-$ denotes an anion of a weakly coordinating acid, such as $BF_4^-$, $ClO_4^-$, $SO_4H^-$, $PF_6^-$, $R^4$-$COO^-$, or $R^4SO_3^-$, where $R^4$ denotes a fluorinated alkyl or aryl group into an α-amino acid derivative of the formula III

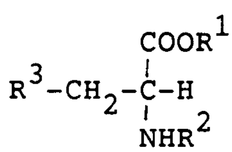

III

in which

- $R^1$ denotes hydrogen or an alkyl group
- $R^2$ denotes a protecting group and
- $R^3$ denotes hydrogen, an alkyl, aryl, alkaryl, aralkyl, heteroalkyl or heteroaryl group

The process according to the invention significantly extends the possibilities of preparing optically active agents, such as pharmaceuticals, herbicides, insecticides or pheromones. Thus, this invention can be used in chemical and pharmaceutical industry.

The asymmetric hydrogenation of amino acid precursors into optically active α-amino acid derivatives is a largely investigated reaction (e.g. Ojima et al., Tetrahedron 1989, 6901) and is also of technological importance (Kagan, Bull. Soc. Chim. Fr. 1988, 846). In general, methanol is preferred as reaction medium to obtain optimal activity and enantioselectivity. Recently proposals have been made to immobilize cationic complex catalysts on cation exchangers (Selke, J. Mol. Cat. 1990, 63, 319) thus obtaining contacts which can also be used in the reaction medium water due to their activity and enantioselectivity.

Another possibility of asymmetric hydrogenation in water which, in contrast to methanol, is not toxic and inflammable is the use of watersoluble phosphines as ligands (Sinou, Bull. Soc. Chim. Fr. 1986, 480; Amrani et al., Organometallics 1989, 8, 542). However, a disadvantage of the above-mentioned procedures is the decreased enantioselectivity in the presence of water (Lecomte et al., J. Organometal. Chem. 1989, 370, 277). Asymmetric hydrogenation in the presence of detergents has not hitherto been described. An indication of increased activity in the presence of sodium dodecyl sulfate in the case of hydrogenation with specific nonchiral catalysts has been reported in a paper by Whitesides (J. Org. Chem. 1981, 46, 2861). A review of the present technological state leads to the conclusion that asymmetric hydrogenation using water as solvent does not provide satisfactory results despite its high experimental expenditure. In the case of the so-called discontinuous phase-transfer catalysis it is typical to use hydrophobic organic cations for

solubilization in organic phases (cf., e.g., J. Blum et al., J. Mol. Catal. 1986, 37, 359). An example for the asymmetric-homogeneous hydrogenation as described below has not hitherto been reported.

The present invention is based on the object of modifying highly active low-molecular enantioselective catalysts for hydrogenation, which could be used efficiently only with alcohols as solvent, so that they can also be used in solvents where their application seemed to be impossible due to low solubility or significant decrease in enantioselectivity.

This object is achieved in accordance with the claims.

The procedure for the preparation of (S)- and (R)-α-amino acid derivatives according to the invention with the formula III

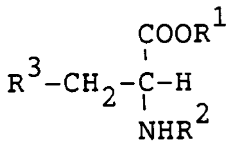

III

in which

- $R^1$ denotes hydrogen or an alkyl group
- $R^2$ denotes a protecting group and
- $R^3$ denotes hydrogen, an alkyl, aryl, alkaryl, aralkyl, heteroalkyl or heteroaryl group

is characterized by the addition of amphiphilic substances, detergents, of the formula IV

A-E IV

in which

- A denotes a nonpolar alkyl chain with 5 to 20 carbon atoms or a glycerol dialkylether group and
- E denotes a polar (hydrophilic)group, such as $-O-SO_3Na$, $-SO_3Na$, $-O(CH_2CH_2O)_nH$ or $-OP^{\ominus}O_2/OCH_2CH_2N^{\oplus}(CH_3)_3/$, where n is a number between 3 and 15.

The hydrogenation may take place in a polar or nonpolar solvent at temperatures between -20 and +60 °C and a normal hydrogen pressure with a molar ratio between detergent and catalyst of 1-100 to 1. Cathionic, anionic, zwitterionic or neutral amphiphilic substances can be used. The addition of detergents is of advantage in molar deficiency with respect to the substrate. Sodium dodecyl sulfate (SDS) and Triton X-100 have proved to be especially suitable detergents. In practice, hydrogenations are carried through in double- walled thermostated vessels under strict exclusion of oxygen and the course of reaction is followed by volumetric measurement of the hydrogen consumption.

As solvent the extremely polar water can be used, which is easily available, nontoxic and inflammable in contrast to alcohols; on the other side, however, even nonpolar solvents, such as toluene, benzene and hexane, can be used. In all cases, high activities and enantionselectivities have been reached. Moreover, aromatic hydrocarbons are of special advantage for hydrophobic substrates.

As catalysts cationic rhodium(I) complexes of the formula II are used

$/Rh(Z)(L_k)/^{+} A^{-}$ II

in which

- Rh denotes rhodium,
- Z denotes an optically active chelating ligand with two trivalent phorphorus atoms as coordinators for the bond to the rhodium,
- L denotes an olefin, diolefin, alcohol or another solvent molecule
- k denotes 0, 1, 2, or 3 and
- A denotes an anion of a weakly coordinating acid, such as $BF_4^-$, $ClO_4^-$, $SO_4H^-$, $PF_6^-$, $CF_3CO_2^-$ and $CF_3SO_3^-$.

Such complexes are for instance described in R. Selke and H. Pracejus, Journal of Molecular Catalysis 37, p.213 (1986).

The molar ratio between catalyst and substrate mostly ranges between 1:100 and 1:3000.

As substrates α-amino acid precurcors of the formula I are used

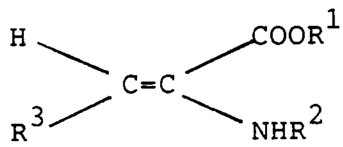

I

in which

- $R^1$ denotes hydrogen or an alkyl group
- $R^2$ denotes a protecting group such as e.g. a $COR^4$-group, where $R^4$ denotes hydrogen, an alkyl, aryl ,aralkyl or alkaryl group and
- $R^3$ denotes hydrogen, an alkyl, aryl, aralkyl, alkaryl, heteroalkyl or heteroaryl group,

Alkyl means within the context of the invention also cyclo-alkyl or alkenyl. The alkyl, aryl, alkaryl, aralkyl, heteroalkyl or heteroaryl groups mostly contain 1-20 C-atoms.

The optically active rhodium(I)-phosphine or rhodium(I)-phosphinite complexes are preferably used as catalysts. As is generally known, if methanol and ethanol are used as solvents, all complexes and substrates investigated react quickly and with high enantioselectivities. The use of water without addition of detergents, however, yields only low enantioselectivities after very long hydrogenation times. An increase in activity and enantioselectivity by the addition of detergents can be observed even in toluene and other aromatic solvents.

The reaction mixture is worked up by shaking the product out with dichloromethane or by filtering the reaction products of low solubility. The catalyst remains mainly in the aqueous phase and can be recycled. The separation of the target product is practically quantitatively feasible by single extraction with dichloromethane.

The activity is determined by the volume-time dependence of hydrogenation while the value for enantioselectivity results from a gas-chromatographic analysis of the dichloromethane extract on a chiral stationary phase. Stepwise extraction and analysis of the single steps revealed that the isolation of the reaction product is not connected with a stereoselection.

In particular, the advantage of the process according to the invention is that the same type of catalyst can be applied both in extremely nonpolar and polar solvents leading to activities and enantioselectivities that have not hitherto been observed in these solvents. Thus, a great number of substrates of highly different solubility can be hydrogenated and worked up in a simple way.

As a result, the former possibilities of preparing optically active substances, such as pharmaceuticals, herbicides, insecticides or pheromones, have been significantly enlarged as the polarity of the substrate can be adopted to the appropriate solvent. Comparing the process according to the invention, the dispersion of the catalyst systems in water using detergents, with the use of watersoluble rhodium phosphine complexes, a stabilization of the catalyst by the detergent toward oxidation and a significant increase in stability have been found.

The following examples shall give a more detailed explanation of the invention.

## Example 1

In a thermostated hydrogenation vessel 9.22 mg (10 µmol) of rhodium-(Z,Z)-cycloocta-1,5-diene/phenyl-2,3-O-bis(diphenyl-phosphino)-β-D-glucopyranoside/-tetrafluoroborate as catalyst precursor and 143.15 mg (1 mmol) of methyl 2-acetamidoacrylate as substrate are suspended in 15 ml of water under anaerobic conditions in the presence of 10 mg (34.7 µmol) of sodium dodecyl sulfate. After stirring for one hour at 25 °C in argon atmosphere the latter is replaced by hydrogen of normal pressure. Hydrogenation is started by stirring at 25 °C and it is finished after 30 min with a half-life time of 8 min. Extraction with dichloromethane (3x5ml) affords (S)-methyl N-acetylalaninate in nearly quantitative chemical yield with an enantioselectivity of 93.4±0.5 % enantiomeric excess (ee) as determined by gas chromatography.

## Example 2

The procedure is as described in example 1, but in the presence of 50 mg (173 µmol) of sodium dodecyl sulfate and at a stirring time reduced to 10 min. Thus, the methyl (S)-N-acetylalaninate is quantitatively obtained with an enantioselectivity of 97.2±0.5 % ee and a half-life time of 2.5 min.

## Example 3

The procedure is as described in example 1, but using 219.2 mg (1 mmol) of (Z)-methyl 2-acetamidocinnamate as substrate. Within 150 min (S)-methyl N-acetyl-phenylalaninate is obtained with an enantioselectivity of 94.4±0.5 % ee and a half-life time of 66 min.

Example 4

The procedure is as described in example 3, but using 50 mg (173 µmol) of sodium dodecyl sulfate. The reaction is completed after 25 min with a half-life time of 6.4 min affording (S)-methyl N-acetyl-phenylalaninate with an enantioselectivity of 97.4±0.5 % ee.

Example 5

The procedure is as described in example 1, but in 15 ml of toluene as solvent. After a hydrogenation time of 5 min with a half-life time of 1.3 min (S)-methyl N-acetylalaninate is obtained with an enantioselectivity of 97.6±0.5 % ee.

Example 6

The procedure is as described in example 5, but using 219.2 mg (1 mmol) of (Z)-methyl 2-acetamidocinnamate as substrate. Within a hydrogenation time of 45 min and with a half-life time of 20.8 min (S)-methyl N-acetylphenylalaninate is obtained with an enantioselectivity of 97.1±0.5 % ee.

Example 7

The procedure is as described in example 6, but decreasing the amount of sodium dodecyl sulfate to 2.5 mg (8.7 µmol). After 50 min and with a half-life time of 32 min (S)-methyl N-acetylphenylalaninate is afforded with an enantioselectivity of 96.4±0.5 % ee.

Example 8

The procedure is as described in example 7, but using only 1 mg (3.5 µmol) of sodium dodecyl sulfate. After 70 min with a half-life time of 41 min this affords (S)-methyl N-acetylphenylalaninate with 95.5±0.5 % ee.

Example 9

The procedure is as described in example 3, but as detergent sodium dodecyl sulfate is replaced by 100 µmol of Triton X-100 which is introduced by adding 1 ml of stock solution (0.304 ml = 323 mg of Triton X-100 to 5 ml of water). Hydrogenation for 60 min with a half-life time of 23 min affords (S)-methyl N-acetylphenylalaninate with an enantioselectivity 95.6±0.5 % ee.

Example 10

The procedure is as described in example 9, but reducing the Triton X-100 amount to 30 µmol (0.3 ml of stock solution). (S)-methyl N-acetylphenylalaninate with an enantio- selectivity of 95.3±0.5 % ee is obtained within 150 min with a half-life time of 65 min.

Example 11

The procedure is as described in example 9, but using exclusively toluene as solvent both for the stock solution of Triton X-100 and for hydrogenation. Within 100 min with a half-life time of 39 min (S)-methyl N-acetylphenylalaninate is obtained with an enantioselectivity of 93.7±0.5 % ee.

Example 12 (comparison example)

The procedure is as described in example 3, but without using detergents. For the complete hydrogenation in water to methyl N-acetylphenylalaninate at least 11 h are required with a half-life time of 6±0.5 h. The enantioselectivity obtained is only 83.5±2 % ee (S)-compound.

Example 13 (comparison example)

The procedure is as described in example 5, but without using detergents. For the complete hydrogenation in toluene to methyl N-acetylalaninate about 40 min are required with a half-life time of 13.4 min. The enantioselectivity obtained is only 90.5±0.5 % ee (S)-compound.

Example 14 (comparison example)

The procedure is as described in example 6, but without using detergents. For the complete hydrogenation about 3 h are required with a half-life time of 1.5±0.5 h. The (S)-methyl N-acetyl-phenylalaninate is obtained with an enantioselectivity of 91.6±0.5 % ee.

Example 15

4.06 mg (10µmol) of /rhodium-bis-(Z,Z)-cycloocta-1,5-diene/tetrafluoroborate and 7.0 mg (11µmol) of (2S,4S)-N-tert.-butoxycarbonyl-4-diphenylphosphino-2-diphenyl-phosphinomethylpyrrolidine are given into a thermostated hydrogenation vessel and the catalyst precursor is prepared in situ by stirring for 15 min in 3 ml abs. methanol under anaerobic conditions. 219.2 mg (1mmol) of (Z)-methyl 2-acetamido-cinnamate, 100 mg (347µmol) of sodium dodecyl sulfate and 15 ml of deaerated water are added. Hydrogenation is initiated by stirring at 25 °C under normal pressure and with a half-life time of 6 min it is completed within 30 min. After extraction with 5 ml of dichloromethane, (R)-methyl N-acetylphenylalaninate is obtained with an enantioselectivity of 95.7±0.5 % ee according to gaschromatographic determination of enantiomers.

Example 16

The procedure is as described in example 15, but at 37°C and increasing the stirring time to one hour. (R)-methyl N-acetylphenylalaninate is obtained quantitatively (half-life time: 12 min) with an enantioselectivity of 96.1±0.5 % ee.

Example 17

The procedure is as described in example 16, but using 50 mg of sodium dodecyl sulfate. With a half-life time of 10 min after one hour (R)-methyl N-acetylphenylalaninate is obtained quantitatively with an enantioselectivity of 93.1±0.5 % ee.

Example 18

The procedure is as described in example 15, but using 28.8 mg (100µmol) of sodium dodecyl sulfate and a solvent ratio of methanol:water = 1:14. With a half-life time of 7 min a complete chemical conversion is obtained after 45 min yielding (R)-methyl N-acetylphenylalaninate with a selectivity of 91.8±0.5 % ee.

Example 19

The procedure is as described in example 18, but using 100 mg (347µmol) of sodium dodecyl sulfate. After 40 min and with a half-life time of 8 min the hydrogenation to (R)-methyl N-acetylphenylalaninate is completed with a selectivity of 95.7±0.5 % ee.

Example 20

The procedure is as described in example 19, but using 150 µl (250µmol) Triton X-100 instead of sodium dodecyl sulfate. After one hour and with a half-life time of 8 min the (R)-methyl N-acetyl-phenylalaninate is obtained quantitatively with a selectivity of 91.0±0.5 % ee.

Example 21

The procedure is as described in example 18, but using 150 µl Carbowax 400 instead of sodium dodecyl sulfate. After 2 h and with a half-life time of 20 min the (R)-methyl N-acetyl- phenylalaninate is obtained quantitatively with a selectivity of 81.5±0.5 % ee.

Example 22

The procedure is as described in example 18, but using 150 mg polyglycol instead of sodium dodecyl sulfate. After one hour and with a half-life time of 7 min the (R)-methyl N-acetylphenylalaninate is obtained quantitatively with a selectivity of 84.0±0.5 % ee.

Example 23

The procedure is as described in example 15, but using 15 ml of water as sole solvent and 150 µl (250 µmol) of Triton X-100 instead of the sodium dodecyl sulfate. After one hour and with a half-life time of 9 min a complete hydrogenation yields the (R)-methyl N-acetylphenylalaninate with a selectivity of 90.8±0.5 % ee.

Example 24

The procedure is as described in example 23, but using 35 µl (60 µmol) of Triton X-100. After 3 h and with a half-life time of 19 min a complete hydrogenation yields the (R)-methyl N-acetylphenylalaninate with a selectivity of 88.2±0.5 % ee.

Example 25

The procedure is as described in example 23, but using 100 mg of sodium dodecyl sulfate. After 35 min with a half-life time of 6 min a complete hydrogenation yields the (R)-methyl N-acetylphenylalaninate with an enantioselectivity of 95.7±0.5 % ee.

Example 26

The procedure is as described in example 23, but using toluene as sole solvent. After 2 h with a half-life time of 20 min a complete hydrogenation yields the (R)-methyl N-acetylphenylalaninate with an enantioselectivity of 93.9±0.5 % ee.

Example 27

The procedure is as described in example 15, but using as ligand 13 mg (11 µmol) (2S,4S)-N-carboxymethyl-di-(dodecyl)-4-diphenylphosphino-2-diphenylphosphino-methylpyrrolidine, as substrate 143.2 mg (1 mmol) of methyl 2-acetamidoacrylate, and 20 mg of sodium dodecyl sulfate. After one hour with a half-life time of 8 min (R)-methyl N-acetylalaninate is obtained with an enantioselectivity of 59.6 % ee.

Example 28

The procedure is as described in example 23, but using 5.5 mg (11 µmol) of (-)-diop as chiral ligand for the catalyst precursor as well as 50 mg of sodium dodecyl sulfate as detergent. After 2 h hydrogenation in water (R)-methyl N-acetylphenylalaninate is obtained with an enantioselectivity of 63.8 % ee with a half-life time of 7 min.

Example 29

The procedure is as described in example 15, but using 150 µl (250 µmol) of Triton X-100 instead of the sodium dodecyl sulfate. (R)-methyl N-acetylphenylalaninate is obtained after 2 h with a half-life time of 8 min and a enantioselectivity of 58.0±1.0 % ee.

Example 30

The procedure is as described in example 28, but using 30 µl (40 µmol) of 1,2-dihexadecylphosphatidylcholine. (R)-methyl N-acetylphenylalaninate is obtained with a half-life time of 16 min with an enantioselectivity of 15.8 % ee.

Example 31

The procedure is as described in example 23, but using 15 ml of n-hexane. A chemical conversion of 75 % is obtained after 4 h yielding (R)-methyl N-acetylphenylalaninate with a half- life time of 75 min and an enantioselectivity of 74.9 % ee.

Example 32

The procedure is as described in example 25, but using 0.40 mg of /Rh(COD)$_2$/BF$_4$ and 0.70 mg of BPPM (cat/sub = 1:1000) and adding 58 mg of sodium dodecyl sulfate. (R)-methyl N-acetylphenylalaninate is obtained quantitatively after 2 h with a half-life time of 7 min and an enantioselectivity of 92.7±0.5 % ee.

Example 33 (comparison example)

The procedure is as described in example 15, but without using any detergents. Hydrogenation is completed after 3 h yielding (R)-methyl N-acetylphenylalaninate with an enantioselectivity of 84.9±1.0 % ee.

Example 34 (comparison example)

The procedure is as described in example 18, but without using any detergents. Hydrogenation is completed after 18 h yielding (R)-methyl N-acetylphenylalaninate with an enantio- selectivity of 80.8±1.0 % ee.

Example 35 (comparison example)

The procedure is as described in example 23, but without using any detergents. After hydrogenation for 18 h (R)-methyl N-acetylphenylalaninate is obtained with an enantioselectivity of 62.2 % ee and a conversion of 38 %. The formation of metallic rhodium can be observed.

Example 36 (comparison example)

The procedure is as described in example 26, but without using any detergents. Hydrogenation in toluene for 5 h affords (R)-methyl N-acetylphenylalaninate quantitatively with an enantioselectivity of 81.1 % ee and a half-life time of 30 min.

Example 37 (comparison example)

The procedure is as described in example 27, but without using any detergents. After hydrogenation for 24 h with a half-life time of 3 h methyl N-acetylphenylalaninate is obtained quantitatively with an enantioselectivity of 33.7 % ee (R)-compound.

Example 38 (comparison example)

The procedure is as described in example 31, but in n-hexane without using detergents. Within a period of 5 h no hydrogen uptake can be observed.

Example 39 (comparison example)

The procedure is as described in examples 28, 29 and 30, but without using any detergents. Within a period of 4 h no hydrogen uptake can be observed.

Example 40

The procedure is as described in example 32, but using a catalyst consisting of 0.33 mg (0.81 µmol) of /Rh(COD)$_2$/BF$_4$ and 0.68 mg (0.97 µmol) of BPPM. Quantitative hydrogenation of 1095 mg (5 mmol) of (Z)-methyl 2-acetamidocinnamate in the presence of 130 mg (200 µmol) of Triton X-100 affords (R)-methyl N-acetylphenylalaninate with an enantioselectivi- ty of 69.2±2 % ee after 5 h with a half-life time of 45 min.

Example 41

The procedure is as described in example 2, but adding 4.6 mg (5 µmol) of the catalyst precursor of example 1 and 58 mg (0.2 mmol) of sodium dodecyl sulfate as detergent. After 90 min 1095 mg (5 mmol) of (Z)-methyl 2-acetamidocinnamate are hydrogenated quantitatively affording (S)-methyl N-acetylphenylalaninate with an enantioselectivity of 95.4±0.5 % ee and a half-life time of 47 min. For preparative work-up the reaction solution was extracted with dichloromethane (3x10 ml) and evaporated to dryness.

Example 42

In a two-necked flask a stock solution of the catalyst complex is prepared by stirring 5.4 mg (13.3 µmol) of /Rh(COD)$_2$/BF$_4$, 9.3 mg (14.7 µmol) of BPPM, and 77.3 mg (270 µmol) of sodium dodecyl sulfate in 20 ml of water under anaerobic conditions. 15 ml of this solution are added to 4.384 g (20 mmol) of (Z)-methyl 2-acetamidocinnamate into a hydrogenation vessel as described in example 1. Hydrogenation of the suspension under stirring for 4 h yields (R)-methyl N-acetylphenylalaninate with a conversion of 93 % and an enantioselectivity of 94±1 % ee. The (S)-component remains completely in the solution which contains only 11 % of the hydrogenation product with 48.5 % ee (R)-compound. The suspended part is filtered and dried yielding 3.946 g. It contains 89 % of the organic substance and consists of 93 % of optically pure (R)-methyl N-acetylphenylalaninate beside 7 % of residual substrate.

Example 43

The procedure is as described in example 2, but using 327 mg (1 mmol) of (Z)-2-benzoylamido-3',4'-dimethoxycinnamic acid as substrate suspended in 15 ml of deaerated water. Hydrogenation for 5 h yields the dopa precursor N-benzoyl-3,4-dimethoxyphenylalanine with a conversion of 97 %. The product which has a low solubility in water can be filtered without further treatment. Washing with water (two times) and subsequent drying yield 320 mg (97 %) with a melting point of 167-169 °C. Enantiomeric separation by HPLC indicates an enantiomeric purity of 93.7 % ee (S) enantiomer.

Example 44

The procedure is as described in example 43, but 2.45 g (7.5 mmol) of the given substrate are suspended in 25 ml of water in the presence of 86.5 mg (0.3 mmol) of sodium dodecyl sulfate and hydrogenated almost quantitatively within 15 h in the presence of 13.8 mg (0.015 mmol) of the given catalyst. The solid obtained in 98 % yield after filtration contains only minor residues of the starting material (m.p. 166 to 179 °C) and has an optical purity of 91.4 % (S), which can be increased by single recrystallisation to 98.2 % ee (S)-N-benzoyl-3,4-dimethoxyphenylalanine (m.p. 176 to 178 °C).

Example 45

The procedure is as described in example 2, but 4.61 g (15 mmol) of (Z)-methyl 2-acetamido-4'-acetoxy-3'-methoxy-cinnamate as substate is suspended in 55 ml of deaerated water and hydrogenated for 5 h at 60 °C in the presence of 27.6 mg (0.03 mmol) of the given catalyst yielding the dopa precursor methyl N-acetyl-4-acetoxy-3-methoxyphenylalaninate quantitatively. The hydrogenation product remains in solution which can be cleared by filtration. The product crystallizes in the cold, is filtered, washed with water, and dried.

Yield 3.5 g (75.4 % of calcd. amount), m.p. 110-111 °C,/$\alpha$/$_D^{25}$ + 79.8° (c = 2.0; CHCl$_3$), enantiomeric purity by GC: 100 ±0.2% ee (S).

Extraction with dichloromethane from the mother liquor affords a second fraction:
Yield 0.94 g (20.3 % of calcd. amount), m.p. 101-105 °C, /$\alpha$/$_D^{25}$ + 48.7° (c = 2.0; CHCl$_3$), enantionmeric purity by GC: 63.5±0.5 % ee (S).

## Claims

1. Process for the preparation of enantiomers of α-amino acid derivatives by asymmetric hydrogenation starting from a substrate of the formula I

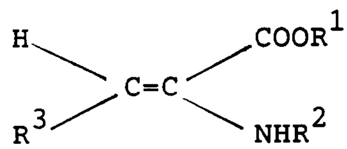

I

in which

$R^1$ denotes hydrogen or an alkyl group

$R^2$ denotes a protecting group and

$R^3$ denotes hydrogen, an alkyl, aryl, aralkyl, alkaryl, heteroalkyl or heteroaryl group,

which is hydrogenated in the presence of a rhodium(I)complex catalyst of the formula II

$/Rh(Z)(L_k)/^{+} A^{-}$ II

in which

Rh denotes rhodium

Z denotes a chiral chelating ligand with two trivalent phosphorous atoms as coordination partners for a bond to rhodium

L denotes a solvent molecule

k denotes 0, 1, 2, or 3 and

$A^-$ denotes an anion of a weakly coordinating acid, such as $BF_4^-$, $ClO_4^-$, $SO_4H^-$, $PF_6^-$, $R^4$-$COO^-$, or $R^4SO_3^-$, where $R^4$ denotes a fluorinated alkyl or aryl group

into an α-amino acid derivative of the formula III

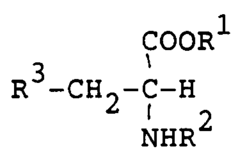

III

in which

$R^1$ denotes hydrogen or an alkyl group

$R^2$ denotes a protecting group and

$R^3$ denotes hydrogen, an alkyl, aryl, aralkyl, alkaryl, heteroalkyl or heteroaryl group

characterized by the addition of an amphiphilic substance of the formula IV

A-E IV

in which

A denotes a nonpolar alkyl chain with 5 to 20 carbon atoms or a glycerol dialkylether group and

E denotes a polar (hydrophilic)group, such as $-O-SO_3Na$, $-SO_3Na$, $-O(CH_2CH_2O)_nH$ or $-OP\ominus O_2/OCH_2CH_2N\oplus(CH_3)_3/$, where n is a number between 3 and 15.

**2.** Process according to claim 1, characterized in that the reaction is carried out at a temperature between -20 and +60 °C.

**3.** Process according to claim 1 or 2, characterized in that the molar ratio between amphiphilic substance and catalyst is 1:1 - 100:1.

**4.** Process according to any of claims 1-3, characterized in that the detergents sodium dodecyl sulfate (SDS), 1,2-dihexadecylphosphatidylcholine, or Triton X-100 are used as amphiphilic substances.

**5.** Process according to any of claims 1-4, characterized in that the amphiphilic substance is used in molar deficiency with respect to the substrate.

**6.** Process according to any of claims 1-5, characterized in that the reaction is carried out in water as

solvent.

**7.** Process according to any of claims 1-5, characterized in that the reaction is carried out in benzene, toluene or hexane as solvents.

**8.** Process according to any of claims 1-7, characterized in that the target product is separated from the aqueous phase by single extraction with dichloromethane.

European Patent Office

# EUROPEAN SEARCH REPORT

Application Number

EP 92 20 0311

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | JOURNAL OF ORGANIC CHEMISTRY, vol. 46, no. 14, 3rd July 1981, pages 2861-2867, Washington, DC, US; R.G. NUZZO et al.: "Synthesis of functional chelating diphosphines containing the bis[2-(diphenylphosphino)ethyl]amino moeity and the use of these materials in the preparation of water-soluble diphosphine complexes of transition metals" * Page 2863, right-hand column * | 1-8 | C 07 C 231/18 |
| A,D | JOURNAL OF ORGANOMETALLIC CHEMISTRY, vol. 370, 1989, pages 277-284, Lausanne, CH; L. LECOMTE et al.: "Chiral sulphonated phosphines. II. Influence of water on the enantioselectivity in the reduction of dehydro-aminoacids" * Whole document * | 1-8 | |
| A,D | ORGANOMETALLICS, vol. 8, no. 2, 1989, pages 542-547, Washington, DC, US; Y. AMRANI et al.: "Chiral sulfonated phosphines. Syntheses and use as ligands in asymmetric hydrogenation using an aqueous-organic two-phase solvent system" * Pages 542-543 * | 1-8 | TECHNICAL FIELDS SEARCHED (Int. Cl.5)<br>C 07 C 231/00 |

The present search report has been drawn up for all claims

| Place of search | Date of completion of the search | Examiner |
|---|---|---|
| THE HAGUE | 21-05-1992 | SANCHEZ Y GARCIA J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document